(19) Europäisches Patentamt / European Patent Office / Office européen des brevets

(11) **EP 1 390 016 B2**

(12) **NEW EUROPEAN PATENT SPECIFICATION**

After opposition procedure

(45) Date of publication and mention
of the opposition decision:
**20.06.2012 Bulletin 2012/25**

(45) Mention of the grant of the patent:
**10.10.2007 Bulletin 2007/41**

(21) Application number: **02757932.5**

(22) Date of filing: **01.04.2002**

(51) Int Cl.:
*A61K 9/14* (2006.01)     *B01F 17/00* (2006.01)
*A61B 8/00* (2006.01)

(86) International application number:
**PCT/US2002/010260**

(87) International publication number:
**WO 2002/078611 (10.10.2002 Gazette 2002/41)**

(54) **ECHOGENIC POLYMER MICROCAPSULES AND NANOCAPSULES AND METHODS FOR PRODUCTION AND USE THEREOF**

ECHOGENE POLYMERMIKROKAPSELN UND NANOKAPSELN UND VERFAHREN ZU IHRER HERSTELLUNG UND VERWENDUNG

MICROCAPSULES ET NANOCAPSULES POLYMÈRES ÉCHOGÉNIQUES ET PROCÉDÉS DE PRODUCTION ET D'UTILISATION

(84) Designated Contracting States:
**AT BE CH CY DE DK ES FI FR GB GR IE IT LI LU MC NL PT SE TR**

(30) Priority: **30.03.2001 US 280412 P**

(43) Date of publication of application:
**25.02.2004 Bulletin 2004/09**

(73) Proprietor: **Drexel University**
**Philadelphia, Pennsylvania 19104 (US)**

(72) Inventors:
• **WHEATLEY, Margaret, A.**
**Media, PA 19063 (US)**
• **EL-SHERIF, Dalia**
**Penn Valley, PA 19072 (US)**

(74) Representative: **Grünecker, Kinkeldey,**
**Stockmair & Schwanhäusser**
**Leopoldstrasse 4**
**80802 München (DE)**

(56) References cited:
US-A- 5 585 112     US-A- 5 676 925
US-A- 5 837 221     US-A- 5 853 698
US-A- 5 955 142     US-A- 6 080 429
US-A- 6 139 818

• **NARAYAN P ET AL: "PREPARATION AND CHARACTERIZATION OF HOLLOW MICROCAPSULES FOR USE ASULTRASOUND CONTRAST AGENTS" POLYMER ENGINEERING & SCIENCE, WILEY & SONS, BOGNOR REGIS, GB, vol. 39, no. 11, November 1999 (1999-11), pages 2242-2255, XP000926890 ISSN: 0032-3888**
• **BJERKNES K ET AL: "Preparation of polymeric microbubbles: Formulation studies and product characterisation" INTERNATIONAL JOURNAL OF PHARMACEUTICS (AMSTERDAM), vol. 158, no. 2, 8 December 1997 (1997-12-08), pages 129-136, XP002321393 ISSN: 0378-5173**
• **IGARTUA M ET AL: "Enhanced immune response after subcutaneous and oral immunization with biodegradable PLGA microspheres" JOURNAL OF CONTROLLED RELEASE, ELSEVIER SCIENCE PUBLISHERS B.V., AMSTERDAM, NL, vol. 56, no. 1-3, 4 December 1998 (1998-12-04), pages 63-73, XP004153920 ISSN: 0168-3659**
• **JAPANESE JOURNAL OF APPLIED PHYSICS, vol. 38, May 1999 (1999-05), pages 3014-3019, XP002321394**
• **D.M.EL-SHERIF AND M.A.WHEATLEY: 'Development of a Novel Method for Synthesis..' J.BIOMED.MATER.RES.66A 2003, pages 347 - 355**
• **D.M.EL-SHERIF AND M.A.WHEATLEY PROCEEDINGS OF THE IEEE27TH ANNUAL NORTHEAST BIOENG.CONFERENCE 2001, pages 45 - 46**

EP 1 390 016 B2

Remarks:
The file contains technical information submitted after the application was filed and not included in this specification

Remarks:
The file contains technical information submitted after the application was filed and not included in this specification

**Description**

Background of the Invention

Ultrasound contrast agents are used routinely in medical diagnostic, as well as industrial, ultrasound. For medical diagnostic purposes, contrast agents are usually gas bubbles, which derive their contrast properties from the large acoustic impedance mismatch between blood and the gas contained therein. Important parameters for the contrast agent include particle size, imaging frequency, density, compressibility, particle behavior (surface tension, internal pressure, bubble-like qualities), and biodistribution and tolerance.

Gas-filled particles are by far the best reflectors. Various bubble-based suspensions with diameters in the 1 to 15 micron range have been developed for use as ultrasound contrast agents. Bubbles of these dimensions have resonance frequencies in the diagnostic ultrasonic range, thus improving their backscatter enhancement capabilities. Sonication has been found to be a reliable and reproducible technique for preparing standardized echo contrast agent solutions containing uniformly small microbubbles. Bubbles generated with this technique typically range in size from 1 to 15 microns in diameter with a mean bubble diameter of 6 microns (Keller et al. 1986. J. Ultrasound Med. 5:493-498). However, the durability of these bubbles in the blood stream has been found to be limited and research continues into new methods for production of microbubbles. Research has also focused on production of hollow microparticles for use as contrast agents wherein the microparticle can be filled with gas and used in ultrasound imaging. These hollow microparticles, however, also have uses as drug delivery agents when associated with drug products. These hollow microparticles can also be associated with an agent which targets selected cells and/or tissues to produce targeted contrast agents and/or targeted drug delivery agents.

Surfactant stabilized microbubble mixtures for use as ultrasound contrast agents are disclosed in US Patent 5,352,436.

WO 9847540 discloses a contrast agent for diagnostic ultrasound and targeted disease imaging and drug delivery comprising a dispersion of a biocompatible azeotropic mixture, which contains a halocarbon.

WO 9421301 discloses an ultrasound agent consisting of a biocompatible oil-in-water emulsion in which the oil phase comprises an oil-soluble gas/fluid or gas precursor.

U.S. Patent 5,637,289, U.S. Patent 5,648,062, U.S. Patent 5,827,502 and U.S. Patent 5,614,169 disclose contrast agents comprising water-soluble, microbubble generating carbohydrate microparticles, admixed with at least 20% of a non-surface active, less water-soluble material, a surfactant or an amphiphillic organic acid. The agent is prepared by dry mixing, or by mixing solutions of components followed by evaporation and micronizing.

U.S. Patent 5,648,095 discloses hollow microcapsules for use in imaging and drug delivery. The hollow microcapsules are made by combining a volatile oil with an aqueous phase including a water soluble material such as starch or a polyethylene glycol conjugate to form a primary emulsion. The primary emulsion then is combined with a second oil to form a secondary emulsion, which is hardened and allows for microcapsules to form around a liquid core of the volatile oil. The volatile oil is then removed by evaporation leaving a hollow microcapsule.

U.S. Patent 5,955,143 discloses hollow polymer microcapsules that are produced by dissolving a film-forming polymer in a volatile non-aqueous solvent, dispersing into the polymer solution finely divided particles of a volatilizable solid core material, inducing formation of a solid polymer coating on the particulate solid core material to produce polymer microcapsules having an encapsulated solid core. This core is then removed to result in hollow microcapsules that can be then filled with gas for contrast imaging.

Narayan, P. et al (Polymer Engineering and Science, November 1999, Vol. 39, No. 11, pages 2242-2255) discloses hollow polymer microcapsules for use as image contrast agents. Hollow areas in the polymer microcapsules are created by encapsulating sublimable solids followed by lyophilization of the sublimable particles.

US 6,139,818 is directed towards echogenic gas- or air-filled polymer microballoons for use as ultrasonic contrast agents. This document discloses that a porous particle can be formed using an emulsion of (1) a polymer dissolved in liquid naphthalene solution and (2) an aqueous solution with an emulsifier, followed by freeze-drying. This leads to forming micropores in an envelope of the microballoons.

US 5,853,698 is directed towards echogenic polymeric microcapsules containing fluorinated gases. This document describes emulsifying a solution of a synthetic polymer with an aqueous solution of a volatile salt pore forming agent or a solid pore forming agent.

Bjerknes, K. et al (International Journal of Pharmaceutics, 158 (1997) 129-136) discloses air-filled polymeric microbubbles for use as ultrasound reflectors. In the method disclosed therein, a polymer is dissolved at high temperature in the same substance that is then removed to form the microbubble core.

US 5,837,221 is directed towards polymer-lipid microencapsulated gases for use as imaging agents, and describes that microparticles formed from the combination of a natural or synthetic polymer and lipid have significantly enhanced echogenicity as compared with microparticles not including the lipid.

El-Sherif and Wheatley 2001 (Bioengineering Conference 2001, Proceedings of the IEEE 27th Annual Northeast

Bioengineering Conference) is directed towards the development of biodegradable PLGA microspheres for use as ultrasound contrast agents, and describes a method for producing hollow/porous PLGA microcapsules by a modified double emulsion, solvent evaporation technique. Small, echogenic microspheres can be prepared by encapsulation of a removable volatile core.

There remains a need for methods of production of biocompatible, biodegradable echogenic microcapsules and nanocapsules of a reproducible size range having enhanced echogenicity that can be used for contrast imaging and/or drug delivery with or without targeting capabilities.

## Summary of the Invention

An object of the present invention is to provide methods for producing echogenic polymer microcapsules and nanocapsules which comprise dissolving a non-water soluble substance which dissolves in a non-polar solvent and sublimes from the solid state, in one or more volatile non-polar solvents to form a first mixture; dissolving a polymer in said first mixture to form a second mixture; emulsifying the second mixture with water to produce a first population of microcapsules comprising the polymer and the non-water soluble substance; mixing said first population of microcapsules with a surfactant solution and stirring/homogenizing to break apart the first population of microcapsules and form a second population of microcapsules and/or nanocapsules that are smaller in size; hardening said second population of microcapsules and/or nanocapsules with a solvent; and washing and preferably freeze drying said second population of microcapsules and/or nanocapsules to remove excess non-water soluble substance from said second population of microcapsules and/or nanocapsules.

Thus, according to a first aspect of the present invention, there is provided a method for producing echogenic polymer microcapsules or nanocapsules, the method comprising:

(a) dissolving a non-water soluble substance which sublimes in one or more volatile non-polar solvents to form a first mixture;

(b) dissolving a polymer in said first mixture to form a second mixture;

(c) adding water comprising a water soluble agent which sublimes to said second mixture prior to emulsifying the second mixture to produce a first population of microcapsules comprising the polymer and the non-water soluble substance;

(d) mixing said first population of microcapsules with a surfactant to break apart the first population of microcapsules and form a second population of microcapsules or nanocapsules that are smaller in size;

(e) hardening said second population of microcapsules or nanocapsules by mixing said microcapsules or nanocapsules with a hardening agent; and

(f) washing said second population of microcapsules or nanocapsules to obtain echogenic polymer microcapsules or nanocapsules.

According to a second aspect of the present invention, there is provided an echogenic polymer microcapsule or nanocapsule obtainable by the methods of the present invention.

According to a third aspect of the present invention, there is provided a contrast agent for diagnostic imaging in a subject which comprises an echogenic polymer microcapsule or nanocapsule of the present invention that is filled with a gas. Such contrast agents may further comprise a targeting agent such as a peptide or antibody on the microcapsule or nanocapsule surface for targeting of the contrast agents to selected tissues or cells. Attachment of a targeting agent selective to a diseased tissue provides for a contrast agent which distinguishes between diseased and normal tissue. Use of contrast agents comprising echogenic polymer nanocapsules of the present invention permits imaging of tissues via access to locations of the vasculature too narrow for access via microcapsules, e.g. leaky tumor vasculature.

Also described herein are methods for imaging a tissue or tissues in a subject via administration of a contrast agent comprising echogenic polymer microcapsules and/or nanocapsules of the present invention that are filled with a gas. Contrast agents used in this method may further comprise a targeting agent such as a peptide or antibody on the microcapsule and/or nanocapsule surface for targeted delivery of the contrast agent to the selected tissue or tissues. Attachment of a targeting agent selective to a diseased tissue provides for a method of distinguishing via selective imaging diseased tissue from normal tissue. Similarly, attachment of a targeting agent selective to a malignant tissues provides for a method of distinguishing via selective imaging malignant tissue from benign tissue.

According to a fourth aspect of the present invention, there is provided a composition for delivery of a bioactive agent which comprises a bioactive agent adsorbed to, attached to, encapsulated in, or any combination thereof, an echogenic polymer microcapsule or nanocapsule of the present invention. Such compositions may further comprise a targeting agent such as a peptide or antibody on the microcapsule or nanocapsule surface for targeting of the bioactive agent to selected tissues or cells. Attachment of a targeting agent selective to a diseased tissue provides for a delivery agent which delivers a bioactive agent selectively to diseased tissue. The bioactive agent can be released from the microcapsule or nanocapsule by exposure to ultrasound and/or upon degradation of the polymer capsule. Use of compositions comprising echogenic polymer nanocapsules of the present invention permits delivery of bioactive agents to locations of the vasculature too narrow for access via microcapsules, e.g. leaky tumor vasculature.

Also described herein are methods for delivery of bioactive agents to a subject via administration of a composition comprising bioactive agent adsorbed to, attached to, and/or encapsulated in, or any combination thereof, echogenic polymer microcapsules and/or nanocapsules of the present invention. Compositions used in this method may further comprise a targeting agent such as a peptide or antibody on the echogenic polymer microcapsule and/or nanocapsule surface for targeting of the bioactive agent to selected tissues or cells in the subject. In this method, bioactive agent is released from the microcapsule and/or nanocapsule by exposure to ultrasound, degradation of the polymer capsule or a combination thereof.

## Detailed Description of the Invention

The present invention relates to echogenic polymer microcapsules and nanocapsules and methods for producing such for use as contrast agents in diagnostic imaging and compositions for delivery of bioactive agents. The present invention further relates to attachment of a targeting agent to the echogenic polymer microcapsules and nanocapsules of the contrast agents and compositions for delivery of bioactive agents of the present invention for targeted diagnostic imaging and delivery of bioactive agents to a selected tissue or tissues. Production of echogenic polymer nanocapsules in accordance with the present invention permits imaging and delivery of bioactive agents to regions of the vasculature too narrow for access via microcapsules.

For purposes of the present invention, by "echogenic" it is meant that the microcapsule or nanocapsule is capable of producing a detectable echo when insonated with ultrasonic waves due to an acoustic impedance mismatch between blood and the microcapsule or nanocapsule. In a preferred embodiment, echogenic characteristics result from the microcapsule and/or nanocapsule being hollow and/or porous. By "porous" for purposes of the present invention, it is meant that the capsules contain one or more pores.

According to the present invention, echogenic polymer microcapsules and/or nanocapsules are produced as follows. A non-water soluble substance which dissolves in a non-polar solvent and sublimes from a solid state is first dissolved in one or more volatile non-polar solvents to form a first mixture. Any non-water soluble substance which dissolves in a non-polar solvent and sublimes from a solid state can be used to produce this first mixture. Examples include but are not limited to, camphor, para-ph, camphene, naphthalene, cocoa butter and theobroma oil. Examples of non-polar solvents which can be used include, but are not limited to, methylene chloride, acetone, acetonitrile, tetrahydrofuran, chloroform, pentane, pentene, and methyl ethyl ketone. In a preferred embodiment, the non-water soluble substance is camphor and the solvent is methylene chloride. A preferred ratio of camphor to methylene chloride is 0.05 grams: 10 ml. Additional non-polar solvents from the above-listed examples can also be added to the mixture to control the size of the capsules in the nano size range (nanocapsules). Addition of a second, preferably different solvent reduces the size of the capsules because of its different properties. For example, the rate at which the solvent leaves the capsules during the hardening phase effects the size of the capsules. Specifically, the faster the solvent leaves the smaller the capsule. Acetone leaves the capsules faster than methylene chloride. Accordingly, addition of acetone as a second solvent results in nanosized capsules in comparison to the microsized capsules formed when using only methylene chloride.

For purposes of the present invention, by "nanocapsule" it is meant a capsule sufficiently small in size to access the microvasculature of the human body. Nanocapsules of the present invention range in size from about 10 nm to about 500 nm, while microcapsules of the present invention range in size from about 500 nm to about 1000 microns. Nanocapsules of this size provide an advantage in that they can access areas difficult if not impossible to reach with microcapsules. For example, nanocapsules can pass through leaky tumor vasculature. In addition, nanocapsules have different resonance frequencies thus providing advantages in both imaging and delivery of bioactive agents. Nanocapsules of the present invention have been found to be echogenic above 10 MHZ.

After the non-water soluble substance is fully dissolved in the non-polar solvent, a polymer material is added to the first mixture. Examples of polymers that can be used in this method include, but are not limited to, polylactide, a polyglycolide, a polycaprolactone, a copolymer of polylactide and polyglycolide, a copolymer of lactide and lactone, a polysaccharide, a polyanhydride, a polystyrene, a polyalkylcyanoacrylate, a polyamide, a polyphosphazene, a poly (methylmethacrylate), a polyurethane, a copolymer of methacrylic acid and acrylic acid, a copolymer of hydroxyethylmethacrylate and methylmethacrylate, a polyaminoacid, and a polypeptide. Preferred polymers are those which are biocompatible and/or biodegradable. In a preferred embodiment the polymer is polylactic co-glycolic acid (PLGA). In this embodiment it is preferred that 0.5 grams of the polymer be added to the 10 ml solution of the first mixture.

The polymer is then stirred into the first mixture until it is completely dissolved, thus forming the second mixture.

The second mixture is then emulsified with water. For emulsification with water, an aliquot of distilled water, preferably 1 ml, is added to a second mixture comprising 10 ml of methylene chloride, camphor and PLGA, and a probe sonicator is used for 30 seconds. This emulsion of a second mixture of methylene chloride, camphor and PLGA produces a population of microcapsules that range in size from 0.1 to 1 mm. In the context of the present invention this population of microcapsules is referred to as the first population of microcapsules.

The first population of microcapsules is then poured into a surfactant and homogenized for several minutes. Examples of surfactant which can be used in the present invention include, but are not limited to, poly(vinyl) alcohol, Tweens and non-ionic surfactants. In a preferred embodiment, microcapsules of camphor and PLGA are added to 50 ml of a 5% polyvinyl alcohol (PVA) solution and homogenized for 5 minutes at 9,500 rpm. The addition of the surfactant allows for the break-up of the microcapsules into smaller beads to produce a second population of microcapsules and/or nanocapsules. This procedure enhances the size reduction of the microcapsules, an important step in the production process. In a preferred embodiment, the second population of microcapsules is poured into a hardening agent such as 100 ml of a 2% isopropanol solution and stirred for one hour. This removes residual methylene chloride and hardens the microcapsules. Alternatively, the isopropanol is poured into the container holding the microcapsules and stirred for one hour to remove residual methylene chloride and to harden the microcapsules. Examples of hardening agents that can be used include, but are not limited to, isopropanol, ethanol, methanol, ethyl ether, petroleum ether, heptane, and hexane.

Following this hardening step, the microcapsules are collected by centrifugation, washed with excess distilled water, centrifuged again, and washed multiple times with a solvent such as hexane which removes the non-water soluble substance without dissolving the polymer. Hexane also acts as a hardening agent removing the residual methylene chloride further drying the capsules. After each wash the wash solvent is removed by pipetting. A final washing with distilled water is then performed. The washed population of polymer microcapsules and/or nanocapsules are then centrifuged, frozen at -85° C, and then lyophilized to dry the capsules and remove any additional residual non-water soluble substance. This results in a free flowing powder of microcapsules and/or nanocapsules that is stable upon storage and can be resuspended routinely in a pharmaceutically acceptable vehicle such as saline just prior to use.

Particle size analysis of the final population of echogenic polymer microcapsules produced from camphor, methylene chloride and PLGA showed the population to be uniform in size with a range of 0.4 to 1.6 microns.

Acoustic dose-response experiments were performed using these echogenic polymer microcapsules. Four single element, broadband, 12.7 mm element diameter, 50.8 mm spherically focused transducers (Panametrics, Waltham, MA) with center frequencies of 2.25 MHZ, 5 MHZ, 7.5 MHZ and 10 MHZ, respectively, were chosen to represent the conventional medical ultrasound range. The 6 dB bandwidths of the transducers were 89%, 92%, 71% and 65% respectively. A known quantity (0.01 g to 1.0 g) of the echogenic polymer microcapsules was weighed into 50 ml of phosphate-buffered saline in a sample container and a dose-response was recorded. Doses on the dose-response curve were repeated six times. Fresh buffer was used at each dose and a 10 second delay post-administration of the agent in the container ensured proper mixing prior to collecting any signal. The signal was 2.0 micro seconds time-gated, after the ringing from the wall signal had subsided. The root mean square (rms) of the gated signal was calculated and the average for 50 A-lines with no agent was taken as baseline $s_0(t)$. Similarly, the average for 50 A-lines with contrast agent $s_{CA}(t)$ was calculated and presented relative to $s_0(t)$ as enhancement ($\Sigma E$) expressed in dB.

$$\Sigma E = 20 \log_{10}[rms(s_{CA}(t))/rms(s_0(t))]$$

The microcapsules of the present invention produced a dose-response relationship.

In the emulsification step, an agent which is dissolved in an aqueous solution and which sublimes is added. Examples of such agents which are water soluble and which sublime include, but are not limited to ammonium carbonate and other ammonium salts, theobromine and theobromine acetate. In a preferred embodiment, 1.0 ml of a 4% ammonium carbonate aqueous solution is added to the second mixture prior to probe sonication.

Size distribution analysis of these microcapsules via Coulter size analysis showed a mean diameter of 1.242 $\mu$m. Size distribution analysis of microcapsules via Horiba size analysis revealed a mean diameter of 1.210 $\mu$m.

The polymer microcapsules prepared in accordance with this method using camphor and ammonium carbonate were echogenic and an in vitro dose response was shown to be related to the frequency at which the capsules were insonated giving an enhancement of 14.8, 25.4, 25.3 and 20.8 dB when insonated with 2.25, 5, 7.5, and 10 MHZ ultrasound energy, respectively, for a dose of only 8 $\mu$g of microcapsules/ml of buffer (approximately 1.6 x $10^6$ micro-

bubbles per ml). This dose is relatively low in comparison to the 6 mg/ml dose required to achieve a 23 dB enhancement from microcapsules wherein only camphor was encapsulated and sublimed. Thus, microcapsules prepared in accordance with this method wherein ammonium carbonate, an agent that is soluble and sublimes, is dissolved in an aqueous solution and added prior to emulsification, require a lower dose to give the same acoustic enhancement as microcapsules prepared without this additional step.

A shadowing effect was also observed with the microcapsules.

In vivo power Doppler imaging was also performed in New Zealand White rabbits using these microcapsules as the imaging agent and showed enhancement of the image in comparison to imaging taken without the agent. *In vivo* dose response curves showed a significant acoustic enhancement of up to 24 dB, with a dose of 0.15 mL/kg.

Thus, as demonstrated herein, echogenic polymer microcapsules and nanocapsules produced in accordance with these methods can be used for imaging of any of the various tissues and epithelium and/or endothelium thereof routinely imaged with ultrasound techniques including, but not limited to, renal tissue, brain tissue, tumor vasculature, skin tissue, pancreatic tissue, breast tissue, heart tissue, prostate tissue, uterine tissue, adrenal gland tissue, retinal tissue, muscle tissue, areas of plaque and areas of ischemia.

For use as a contrast agent, it is preferred that the echogenic microcapsules and/or nanocapsules of the present invention be hollow or porous so that they can be filled with gas. Such gas-filled polymer microcapsules are produced by introducing echogenic hollow or porous polymer microcapsules into contact with a gas and equilibrating the microcapsules with the gas for a period of time sufficient to allow diffusion of the gas into the polymer microcapsules, resulting in a gas-filled polymer microcapsule. This procedure of exposing hollow or porous polymer microcapsules to the gas may be carried out at ambient pressure (atmospheric), at subatmospheric pressure, or at an elevated pressure. The period of time required to effect filling of the hollow microcapsules with the gas is relatively short, typically requiring only a few minutes, the actual time depending on the manner and pressure at which the hollow microcapsules are equilibrated with the gas. The term "gas" as used in this specification includes substances which are in gaseous form under normal storage conditions, *e.g.,* at about 15 to 25° C, and/or at normal mammalian body temperature, *e.g.,* 37° C in humans. The resulting gas-filled polymer microcapsules of this invention may be stored as a dry, free-flowing powder, preferably in the presence of the gas contained in the polymer microcapsules.

The gas-filled microcapsules are useful as contrast agents in medical imaging, such as diagnostic ultrasound. Ultrasound contrast compositions typically comprise the hollow or porous polymer microcapsules, filled with a gas, and dispersed in an aqueous liquid which serves as a carrier for the contrast agent. Aqueous liquids that can be used include, but are not limited to, isotonic saline and phosphate-buffered saline. The contrast agent composition is then injected into the bloodstream and used for ultrasound visualization of specific blood vessels or body organs.

The polymer microcapsules and nanocapsules of the present invention can be used for delivery of bioactive agents. In this embodiment, a bioactive agent may be adsorbed to and/or attached to the surface of the microcapsule or nanocapsule. To adsorb a drug product to the microcapsule surfaces, the drug is dissolved in distilled water or a buffer, and then the dried microcapsules are suspended in distilled water with the drug. The suspension is stirred overnight and then the suspension centrifuged to collect capsules. The resulting microcapsules are then washed, frozen and lyophilized. The lyophilized microcapsules have the drug product to be delivered adsorbed to their surfaces. Bioactive agents can also be attached to the microcapsules and/or nanocapsules in accordance with well known methods for conjugation. For example, a conjugation method such as taught in Example 8 may be used substituting the bioactive agent for the RGD peptide. Alternatively, or in addition, a bioactive agent can be encapsulated in the microcapsule or nanocapsule. Water soluble bioactive agents can be encapsulated in the microcapsules or nanocapsules by including water during emulsification and dissolving the bioactive agent in this water. Non-water soluble bioactive agents can be encapsulated in the microcapsules or nanocapsules by dissolving the bioactive compound in the non-polar organic solvent in the first step of preparation of these capsules. Examples of bioactive agents which can be adsorbed, attached and/or encapsulated in the microcapsules and/or nanocapsules of the present invention include, but are not limited to, antineoplastic and anticancer agents such as azacitidine, cytarabine, fluorouracil, mercaptopurine, methotrexate, thioguanine, bleomycin peptide antibiotics, podophyllin alkaloids such as etoposide, VP-16, teniposide, and VM-26, plant alkaloids such as vincristine, vinblastin and paclitaxel, alkylating agents such as busulfan, cyclophosphamide, mechlorethamine, melphanlan, and thiotepa, antibiotics such as dactinomycin, daunorubicin, plicamycin and mitomycin, cisplatin and nitrosoureases such as BCNU, CCNU and methyl-CCNU, anti-VEGF molecules, gene therapy vectors and peptide inhibitors such as MMP-2 and MMP-9, which when localized to tumors prevent tumor growth.

Once prepared, microcapsules and/or nanocapsules comprising the bioactive agent can be suspended in a pharmaceutically acceptable vehicle for injection into animals, including humans. Once injected, the bioactive agent is released by either biodegradation over time of the polymer microcapsule structure, by initiation of release of the bioactive agent through exposure to ultrasound, or by a combination thereof.

Compositions of the present invention can be used to direct delivery of a bioactive agent to any of the various tissues and epithelium and/or endothelium thereof including, but not limited to, renal tissue, lung tissue, brain tissue, tumor vasculature, skin tissue, pancreatic tissue, breast tissue, heart tissue, prostate tissue, intestinal tissue, uterine tissue, adrenal gland tissue, retinal tissue, muscle tissue, areas of plaque, areas of inflammation, and areas of ischemia.

The microcapsules and/or nanocapsules of the present invention may further comprise a targeting agent attached to the capsule surface which upon systemic administration can target the contrast agent or the delivery agent to a selected tissue or tissues, or cell in the body. Targeting agents useful in the present invention may comprise peptides, antibodies, antibody fragments, or cell surface receptor-specific ligands that are selective for a tissue or cell. Examples include, but are in no way limited to, RGD which binds to $\alpha v$ integrin on tumor blood vessels, NGR motifs which bind to aminopeptidase N on tumor blood vessels and ScFvc which binds to the EBD domain of fibronectin. Accordingly, targeting agents can be routinely selected so that a contrast agent or delivery agent of the present invention, or a combination thereof, is directed to a desired location in the body such as selected tissue or tissue, cells or an organ, or so that the contrast agent or delivery agent of the present invention can distinguish between various tissues such as diseased tissue versus normal tissue or malignant tissue versus benign tissue. Targeted contrast and/or delivery agents can be administered alone or with populations of contrast agents and/or delivery agents of the present invention which do not further comprise a targeting agent.

RGD peptide was conjugated to echogenic polymer microcapsules of the present invention. The microcapsules were coated with an RGD peptide that targets integrins specific to angiogenesis, $\alpha v\beta 3$ and $\alpha v\beta 5$. The microcapsules were then bound to rat neuroblastoma cells in vitro within 6 hours. The results of this study demonstrate that microspheres bound to a selected targeting agent can be used to target selected cells. Such microcapsules are useful for targeted imaging, targeted therapeutic imaging and delivery of bioactive agents using the microspheres of the present invention as the vehicle.

The following nonlimiting examples are provided to further illustrate the present invention.

## EXAMPLES

### Example 1: Materials

Poly (D,L-lactide-co-glycolic acid 50:50, PLGA) (Medisorb 5050 DL 3A, lot 1010-412) was purchased from Alkermes. Poly (vinyl alcohol) (PVA), 88% mole hydrolyzed, with a $M_w$ of 25,000 was purchased from Polysciences, Inc. (1R)-(+)-Camphor, Ammonium Dihydrogen Phosphate, GRGDS (Gly-Arg-Gly-Asp-Ser) peptide complex, EDC (1-Ethyl-3,-3-Dimethylamino-Propyl) carbodiimide, NHS (N-Hydroxysulfosuccinimide), Antibiotics (penicillin and streptomycin), and L-glutamine were from Sigma. Dulbecco's Modified Eagle Medium (DMEM), Hank's Balanced Salt Solution, and Fetal Bovine Serum (FBS) were purchased from Fisher. Ammonium Carbonate was purchased from J.T.Baker. All other chemicals were reagent grade from Fisher.

### Comparative Example 2: Production of camphor containing microcapsules

Camphor (0.05 g) and PLGA (0.5 g) were dissolved in 10 ml of methylene chloride and probe sonicated at 117 Volts for 30 seconds. The resulting emulsion was then poured into a 5% PVA solution and homogenized for 5 minutes at 9,500 rmp. The homogenate was then poured into a 2% isopropanol solution and stirred for 1 hour. The capsules were collected by centrifugation, washed three times with hexane, then once with deionized water and lyophilized, using a Virtis Benchtop freeze dryer, to remove the camphor core.

### Example 3: Preparation of camphor and ammonium carbonate containing microcapsules

Camphor (0.05 g) and PLGA (0.5 g) were dissolved in 10 ml of methylene chloride. 1.0 ml of 4% ammonium carbonate solution was added to the polymer solution and probe sonicated at 117 Volts for 30 seconds. The emulsion was then poured into a 5% PVA solution and homogenized for 5 minutes at 9,500 rmp. The homogenate was then poured into a 2% isopropanol solution and stirred for 1 hour. The capsules were collected by centrifugation, washed three times with hexane, then once with deionized water and lyophilized, using a Virtis Benchtop freeze dryer, to remove the camphor and ammonium carbonate core.

### Example 4: Size Distribution

Static light scattering using a Horiba LA-910 Particle Size Analyzer (Horiba Instrument) and Coulter Multisizer II were used and compared to measure the size distribution of the capsules.

### Example 5: Scanning Electron Microscopy (SEM)

Scanning electron micrographs, (using an Amray model 1830D SEM) were taken of the freeze dried capsules.

The capsules were mounted on metal stubs with double sided electrical tape. They were gold coated and viewed under the SEM.

**Example 6: *In Vitro* Acoustic Studies**

The freeze-dried capsules were weighed into a 100 ml custom-made vessel equipped with an acoustic window, and 50 ml of phosphate buffered saline (PBS) solutions were added. The suspension was then placed in the acoustic set-up and sonicated using transducers with varying center frequencies, 2.25, 5, 7.5 and 10 MHZ. The acoustic enhancement was analyzed using Lab View. Dose response curves were constructed for doses in the range of 0 mg/ml to 12 $\mu$g/ml, from readings taken within the first 30 seconds.

**Example 7: *In Vivo* Acoustic Studies**

All in vivo studies were performed on New Zealand white rabbits of either sex, within the weight range of 2.0 to 5.0 kg. The rabbits were sedated with an intramuscular injection of 0.65 mg/ml of Ketamine hydrochloride (Ketaset, Aveco) and xylazine hydrochloride (Gemimi). After each experiment the rabbit was sacrificed by a lethal dose of pentobarbital (Beuthanasia). The agent was weighed and suspended in saline prior to injection. The agent was injected through a catheterized ear vein using an 18-gauge needle. Immediately after injection the injection port was flushed with 5 cc saline. Power Doppler imaging of the kidney was performed using an abdominal scan.

*In vivo* dose response curves were constructed in different rabbits. A custom made Silex 10 MHZ cuff transducer was placed around the distal aorta below the renal arteries, which was exposed surgically prior to the experiment. This setup reduced interference and noise from respiratory movements. A pulsed Doppler instrument (SDD 600; Vingmed Sound, Oslo, Norway) recorded the quadrate audio outputs. The contrast enhancement (in dB) was calculated by a known technique from the recorded data, the change in power over time.

**Example 8: Coating of microcapsules with RGD peptide**

Dried microcapsules (100 mg) were combined with 5 mg 1-ethyl-3-(dimethylamino-propyl)-carbodiimide (EDC) (1:1 mole ratio of COOH groups), 1.4 mg of N-hydroxysuccinimide (NHS) (1:2 mole ratio to EDC), in 10 ml of buffer (0.1M MES, 0.3M NaCl, pH 6.5) and stirred for 15 minutes. RGD peptide (150$\mu$g) was then added and stirred for 24 hours. The microcapsules were washed with deionized water three times and lyophilized.

**Example 9: Cell Culture**

The NB2A mouse neuroblastoma cells were cultured using growth medium containing 90% DMEM, 10% FBS, and 2mM L-glutamine. The medium was changed and the cells were split every three days. The experiment was performed on the cells at passage 10.

**Example 10: Microcapsule Attachment**

Cells were plated in each well of a 12-well cell culture plate along with 3 ml of growth medium. After three days, the cells became confluent and the growth medium was changed. The cells were then washed with Hank's salt solution and replaced with a modified medium containing microcapsules, either PLGA with or without RGD peptide, suspended in the growth medium at a concentration of 0.5 mg/ml. The cells were then incubated for 0, 1, and 6 hours. After each specified time point, the medium was removed and the cells were washed again with Hank's salt solution.

The cells were then viewed under a Wesco Verta 7000 series microscope. Digital pictures were taken using an Olympus DP11 digital camera interfaced with the microscope at a magnification of 625x.

**Claims**

1.  A method for producing echogenic polymer microcapsules or nanocapsules, the method comprising:

    (a) dissolving a non-water soluble substance which sublimes in one or more volatile nonpolar solvents to form a first mixture;
    (b) dissolving a polymer in said first mixture to form a second mixture;
    (c) adding water comprising a water soluble agent which sublimes to said second mixture prior to emulsifying the second mixture to produce a first population of microcapsules comprising the polymer and the non-water soluble substance;

(d) mixing said first population of microcapsules with a surfactant to break apart the first population of microcapsules and form a second population of microcapsules or nanocapsules that are smaller in size;

(e) hardening said second population of microcapsules or nanocapsules by mixing said microcapsules or nanocapsules with a hardening agent; and

(f) washing said second population of microcapsules or nanocapsules to obtain echogenic polymer microcapsules or nanocapsules.

**2.** A method according to claim 1, further comprising the step of lyophilizing the echogenic polymer microcapsules or nanocapsules after the washing step.

**3.** An echogenic polymer microcapsule or nanocapsule obtainable by a method according to claim 1 or 2.

**4.** An echogenic polymer microcapsule or nanocapsule according to claim 3, wherein the non water soluble polymer is a member selected from the group consisting of polylactide, polyglycolide, a copolymer of lactide and lactone, a polyanhydride, a polystyrene, a polyalkylcyanoacrylate, a polyamide, a polyphosphazene, a poly(methylmethacrylate), a polyurethane, a copolymer of methacrylic acid and acrylic acid, a copolymer of hydroxyethylmethacrylate and methylmethacrylate, and a polypeptide.

**5.** A contrast agent for diagnostic imaging in a patient, the contrast agent comprising an echogenic polymer microcapsule or nanocapsule according to claim 3 or 4 filled with a gas.

**6.** A contrast agent according to claim 5, further comprising a targeting agent attached to an outer surface of the microcapsule or nanocapsule.

**7.** A contrast agent according to claim 6, which selectively targets diseased tissue and distinguishes the diseased tissue from normal tissue, or which selectively targets malignant tissue and distinguishes the malignant tissue from benign tissue.

**8.** A contrast agent for diagnostic imaging in a subject having tissues with vasculature too narrow for access by microcapsules, the contrast agent according to any of claims 5 to 7 comprising an echogenic nanocapsule.

**9.** A composition for delivery of a bioactive agent, the composition comprising an echogenic polymer microcapsule or nanocapsule according to claim 3 or 4 and a bioactive agent adsorbed to, attached to, encapsulated in, or any combination thereof, the echogenic polymer microcapsule or nanocapsule.

**10.** A composition according to claim 9, further comprising a targeting agent attached to an outer surface of the microcapsule or nanocapsule.

**11.** A composition according to claim 9 or 10, wherein release of the bioactive agent in the subject can be triggered by ultrasound.

**12.** A composition according to claim 9 or 10, wherein the bioactive agent is released by degradation of the echogenic polymer microcapsules and nanocapsules.

**13.** A composition according to claim 12, wherein degradation of the echogenic polymer microcapsules and nanocapsules and release of the bioactive agent is altered by ultrasound.

**14.** A composition according to any of claims 10 to 13, wherein the composition is targeted to diseased tissue or to malignant tissue.

**15.** A composition for delivering a bioactive agent to tissues in a subject having vasculature too narrow for access by microcapsules, the composition according to any of claims 9 to 14 comprising an echogenic polymer nanocapsule.

**Patentansprüche**

**1.** Verfahren zur Herstellung echogener Polymer-Mikrokapseln oder -Nanokapseln, wobei das Verfahren umfasst:

(a) Lösen einer nicht-wasserlöslichen Substanz, welche sublimiert, in einem oder mehreren flüchtigen nichtpolaren Lösungsmittel(n), um eine erste Mischung zu bilden;

(b) Lösen eines Polymers in der ersten Mischung, um eine zweite Mischung zu bilden;

(c) Zugeben von Wasser, das ein wasserlösliches Agens umfasst, welches sublimiert, zu der zweiten Mischung vor dem Emulgieren der zweiten Mischung, um eine erste Population von Mikrokapseln, welche das Polymer und die nichtwasserlösliche Substanz umfassen, herzustellen;

(d) Mischen der ersten Population von Mikrokapseln mit einem Tensid, um die erste Population von Mikrokapseln aufzubrechen und eine zweite Population von Mikrokapseln oder Nanokapseln zu bilden, die eine geringere Größe aufweisen;

(e) Härten der zweiten Population von Mikrokapseln oder Nanokapseln durch Mischen der Mikrokapseln oder Nanokapseln mit einem Härter; und

(f) Waschen der zweiten Population von Mikrokapseln oder Nanokapseln, um echogene Polymer-Mikrokapseln oder -Nanokapseln zu erhalten.

2. Verfahren nach Anspruch 1, welches ferner den Schritt des Lyophilisierens der echogenen Polymer-Mikrokapseln oder -Nanokapseln nach dem Waschschritt umfasst.

3. Echogene Polymer-Mikrokapsel oder -Nanokapsel, erhältlich durch ein Verfahren nach irgendeinem der Ansprüche 1 oder 2.

4. Echogene Polymer-Mikrokapsel oder -Nanokapsel nach Anspruch 3, wobei das nichtwasserlösliche Polymer ein Vertreter ist, welcher aus der Gruppe ausgewählt ist, die aus Polylactid, Polyglykolid, einem Copolymer von Lactid und Lacton, einem Polyanhydrid, einem Polystyrol, einen Polyalkylcyanoacrylat, einem Polyamid, einen Polyphosphazen, einem Poly(methylmethacrylat), einem Polyurethan, einen Copolymer von Methacrylsäure und Acrylsäure, einem Copolymer von Hydroxyethylmethacrylat und Methylmethacrylat sowie einem Polypeptid besteht.

5. Kontrastmittel zur diagnostischen Bildgebung bei einem Patienten, wobei das Kontrastmittel eine echogene Polymer-Mikrokapsel oder -Nanokapsel nach Anspruch 3 oder 4, gefüllt mit einem Gas, umfasst.

6. Kontrastmittel nach Anspruch 5, welches ferner ein zielsuchendes Agens umfasst, das mit einer äußeren Oberfläche der Mikrokapsel oder Nanokapsel verbunden ist.

7. Kontrastmittel nach Anspruch 6, welches selektiv erkranktes Gewebe als Ziel hat und das erkrankte Gewebe von normalem Gewebe unterscheidet oder welches selektiv malignes Gewebe als Ziel hat und das maligne Gewebe von gutartigem Gewebe unterscheidet.

8. Kontrastmittel zur diagnostischen Bildgebung bei einem Individuum mit Geweben mit einem Gefäßsystem, das zu eng für den Zugang durch Mikrokapseln ist, wobei das Kontrastmittel nach irgendeinem der Ansprüche 5 bis 7 eine echogene Nanokapsel umfasst.

9. Zusammensetzung zur Abgabe eines bioaktiven Agens, wobei die Zusammensetzung eine echogene Polymer-Mikrokapsel oder -Nanokapsel nach Anspruch 3 oder 4 und ein bioaktives Agens umfasst, das an die echogene Polymer-Mikrokapsel oder -Nanokapsel adsorbiert, damit verbunden, darin verkapselt oder in irgendeiner Kombination davon vorliegt.

10. Zusammensetzung nach Anspruch 9, welche ferner ein zielsuchendes Agens, verbunden mit einer äußeren Oberfläche der Mikrokapsel oder Nanokapsel, umfasst.

11. Zusammensetzung nach Anspruch 9 oder 10, wobei die Freisetzung des bioaktiven Agens in dem Individuum durch Ultraschall induziert werden kann.

12. Zusammensetzung nach Anspruch 9 oder 10, wobei das bioaktive Agens durch Abbau der echogenen Polymer-Mikrokapseln und -Nanokapseln freigesetzt wird.

13. Zusammensetzung nach Anspruch 12, wobei der Abbau der echogenen Polymer-Mikrokapseln und -Nanokapseln und die Freisetzung des bioaktiven Agens durch Ultraschall verändert wird.

14. Zusammensetzung nach irgendeinem der Ansprüche 10 bis 13, wobei die Zusammensetzung erkranktes Gewebe oder malignes Gewebe als Ziel hat.

15. Zusammensetzung zur Abgabe eines bioaktiven Agens an Gewebe in einem Individuum mit einem Gefäßsystem, welches für den Zugang durch Mikrokapseln zu eng ist, wobei die Zusammensetzung nach irgendeinem der Ansprüche 9 bis 14 eine echogene Polymer-Nanokapsel umfasst.

**Revendications**

1. Procédé de production de microcapsules ou nanocapsules polymères échogènes, le procédé comprenant :

   (a) la dissolution d'une substance non soluble dans l'eau qui se sublime dans un ou plusieurs solvants non polaires volatils pour former un premier mélange ;
   (b) la dissolution d'un polymère dans ledit premier mélange pour former un second mélange ;
   (c) l'addition audit second mélange d'eau comprenant un agent soluble dans l'eau qui se sublime, avant l'émulsification du second mélange pour produire une première population de microcapsules comprenant le polymère et la substance non soluble dans l'eau ;
   (d) le mélange de ladite première population de microcapsules avec un surfactant pour séparer la première population de microcapsules et former une seconde population de microcapsules ou nanocapsules qui sont de taille plus petite ;
   (e) le durcissement de ladite seconde population de microcapsules ou nanocapsules par mélange desdites microcapsules ou nanocapsules avec un agent durcissant ; et
   (f) le lavage de ladite seconde population de microcapsules ou nanocapsules pour obtenir des microcapsules ou nanocapsules polymères échogènes.

2. Procédé selon la revendication 1, comprenant en outre l'étape consistant à lyophiliser les microcapsules ou nanocapsules polymères échogènes après l'étape de lavage.

3. Microcapsule ou nanocapsule polymère échogène pouvant être obtenue par un procédé selon la revendication 1 ou 2.

4. Microcapsule ou nanocapsule polymère échogène selon la revendication 3, dans laquelle le polymère non soluble dans l'eau est un élément choisi dans le groupe constitué par le polylactide, le polyglycolide, un copolymère de lactide et de lactone, un polyanhydride, un polystyrène, un polyalkylcyanoacrylate, un polyamide, un polyphosphazène, un poly(méthylméthacrylate), a polyuréthane, un copolymère d'acide méthacrylique et d'acide acrylique, un copolymère d'hydroxyéthylméthacrylate et de méthylméthacrylate, et un polypeptide.

5. Agent de contraste pour l'imagerie diagnostique chez un patient, l'agent de contraste comprenant une microcapsule ou nanocapsule polymère échogène selon la revendication 3 ou 4 remplie d'un gaz.

6. Agent de contraste selon la revendication 5, comprenant en outre un agent de ciblage fixé à une surface extérieure de la microcapsule ou de la nanocapsule.

7. Agent de contraste selon la revendication 6, qui cible sélectivement le tissu malade et différencie le tissu malade du tissu normal, ou qui cible sélectivement le tissu malin et différencie le tissu malin du tissu bénin.

8. Agent de contraste pour l'imagerie diagnostique chez un sujet ayant des tissus avec un système vasculaire trop étroit pour que des microcapsules y accèdent, l'agent de contraste selon l'une quelconque des revendications 5 à 7 comprenant une nanocapsule échogène.

9. Composition pour la délivrance d'un agent bioactif, la composition comprenant une microcapsule ou nanocapsule polymère échogène selon la revendication 3 ou 4 et un agent bioactif adsorbé à, fixé à, encapsulé dans, ou toute combinaison de ceux-ci, la microcapsule ou nanocapsule polymère échogène.

10. Composition selon la revendication 9, comprenant en outre un agent de ciblage fixé à une surface extérieure de la microcapsule ou de la nanocapsule.

11. Composition selon la revendication 9 ou 10, dans laquelle la libération de l'agent bioactif dans le sujet peut être déclenchée par ultrasons.

**12.** Composition selon la revendication 9 ou 10, dans laquelle l'agent bioactif est libéré par dégradation des microcapsules et nanocapsules polymères échogènes.

**13.** Composition selon la revendication 12, dans laquelle la dégradation des microcapsules et nanocapsules polymères échogènes et la libération de l'agent bioactif sont modifiées par ultrasons.

**14.** Composition selon l'une quelconque des revendications 10 à 13, dans laquelle la composition cible le tissu malade ou le tissu malin.

**15.** Composition pour la délivrance d'un agent bioactif à des tissus chez un sujet ayant un système vasculaire trop étroit pour que des microcapsules y accèdent, la composition selon l'une quelconque des revendications 9 à 14 comprenant une nanocapsule polymère échogène.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5352436 A
- WO 9847540 A
- WO 9421301 A
- US 5637289 A
- US 5648062 A
- US 5827502 A
- US 5614169 A
- US 5648095 A
- US 5955143 A
- US 6139818 A
- US 5853698 A
- US 5837221 A

### Non-patent literature cited in the description

- **Keller et al.** *J. Ultrasound Med.,* 1986, vol. 5, 493-498
- **Narayan, P. et al.** *Polymer Engineering and Science,* November 1999, vol. 39 (11), 2242-2255
- **Bjerknes, K. et al.** *International Journal of Pharmaceutics,* 1997, vol. 158, 129-136